# FASCICULE DE BREVET EUROPEEN

(11) **EP 0 656 354 B1**
(45) Date de publication et mention de la délivrance du brevet: **04.06.1997**
(21) Numéro de dépôt: 94402717.6
(22) Date de dépôt: 29.11.1994
(51) Int. Cl.: C07D 231/14, A61K 31/415

(54) **N-pipéridino-3-pyrazolecarboxamide substitué**
Substituiertes N-Piperidin 3-Pyrazolcarboxamid
Substituted N-piperidino 3-pyrazolecarboxamide

(30) Priorité: 02.12.1993 FR 9314444; 20.07.1994 FR 9408974
(43) Date de publication de la demande: 07.06.1995
(73) Titulaire: SANOFI, 75008 Paris (FR)
(72) Inventeur: Barth, Francis, Rés. Saint James Apt B17, F-34080 Montpellier (FR); Casellas, Pierre, F-34000 Montpellier (FR); Congy, Christian, F-34980 Saint Gely du Fesc (FR); Martinez, Serge, F-34000 Montpellier (FR); Rinaldi, Murielle, F-34680 Saint Georges d'Orques (FR); Anne-Archard, Gilles, F-31500 Toulouse (FR)
(74) Mandataire: Gillard, Marie-Louise

(56) Documents cités:
- EP-A- 0 445 781
- EP-A- 0 477 049
- EP-A- 0 576 357
- US-A- 3 449 350

## Description

La présente invention concerne un nouveau dérivé du pyrazole et ses sels, un procédé pour sa préparation et des compositions pharmaceutiques le contenant.

De nombreux dérivés du pyrazole ont été décrits dans la littérature ; plus particulièrement EP-A-268554 et DE-A-3910248 revendiquent des pyrazoles possédant des propriétés herbicides, EP-A-430186 et JP-A-3031840 revendiquent des composés utiles pour la photographie et EP-A-418845 revendique des pyrazoles pourvus d'activité antiinflammatoire, analgésique et antithrombotique.

EP-A-477 049 décrit des dérivés du pyrazole capables de se lier au récepteur de la neurotensine substitués en position 3 du pyrazole par un groupe 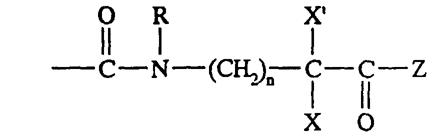 dans lequel notamment R représente l'hydrogène ou un (C₁-C₄)alkyle ; n vaut 0 à 3 ; X et X' forment avec l'atome de carbone quel ils sont liés un groupe pipéridinyl-4 éventuellement N-substitué par un groupe benzyle ; et Z représente un hydroxy, un (C₁-C₆)alcoxy ou un groupe -OPg (Pg = groupe protecteur des acides carboxyliques).

On a maintenant trouvé qu'un N-pipéridino-3-pyrazolecarboxamide possède une très bonne affinité pour le récepteur des cannabinoïdes et est utile dans les domaines thérapeutiques où le cannabis est connu pour intervenir.

Le Δ⁹-THC est le principal constituant actif extrait de *Cannabis sativa* (Tuner, 1985 ; In Marijuana 84, Ed. Harvey, DY, IRL Press, Oxford).

Les effets des cannabinoïdes sont dûs à une intéraction avec des récepteurs spécifiques de haute affinité présents au niveau central (Devane et al., Molecular Pharmacology, 1988, 34, 605-613) et périphérique (Nye et al., The Journal of Pharmacology and Experimental Therapeutics, 1985, 234, 784-791 ; Kaminski et al., 1992, Molecular Pharmacology, 42, 736-742 ; Munro et al., Nature, 1993, 365, 61-65).

La caractérisation de ce récepteur a été rendue possible par la mise au point de ligands synthétiques spécifiques tels que les agonistes WIN 55212-2 (J. Pharmacol. Exp. Ther., 1993, 264, 1352-1363) ou le CP 55,940 (J. Pharmacol. Exp. Ther., 1988, 247, 1046-1051).

Selon un de ses aspects, la présente invention concerne le N-pipéridino-5-(4-chlorophényl)-1-(2,4-dichlorophényl)-4-méthylpyrazole-3-carboxamide de formule : 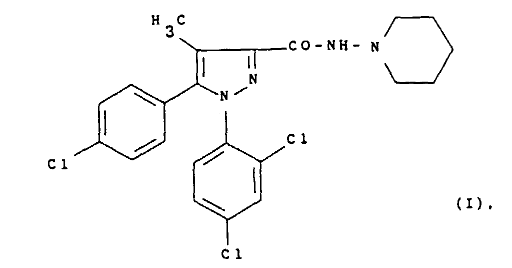 ses sels pharmaceutiquement acceptables et leurs solvates.

Les sels pharmaceutiquement acceptables du composé de formule (I) comprennent les sels d'addition d'acides tels que le chlorhydrate, le bromhydrate, le sulfate l'hydrogénosulfate, le dihydrogénophosphate, le méthanesulfonate, le méthylsulfate, l'oxalate, le maléate, le fumarate, le 2-naphtalènesulfonate, le glyconate, le gluconate, le citrate, l'iséthionate, le paratoluènesulfonate.

Selon un autre de ses aspects, la présente invention concerne un procédé pour la préparation du composé (I) ci-dessus, de ses sels et de leurs solvates, caractérisé en ce qu'on traite un dérivé fonctionnel de l'acide 5-(4-chlorophényl)-1-(2,4-dichlorophényl)-4-méthylpyrazole-3-carboxylique de formule : 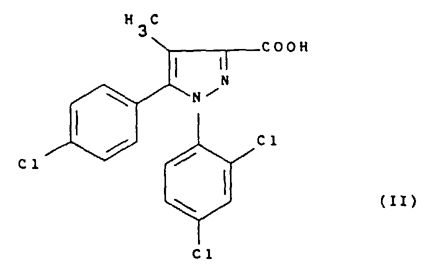 avec la 1-aminopipéridine dans un solvant organique et en présence d'une base, et éventuellement on transforme le composé ainsi obtenu en l'un de ses sels ou l'un de leurs solvates.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-N-oxotris(diméthylamino)phosphonium (BOP).

Ainsi dans le procédé selon l'invention, on peut faire réagir le chlorure de l'acide 5-(4-chlorophényl)-1(2,4-dichlorophényl)-4-méthylpyrazole-3-carboxylique, obtenu par réaction du chlorure de thionyle sur l'acide de formule (II), avec la 1-aminopipéridine, dans un solvant tel que le dichlorométhane, sous une atmosphère inerte, à une température comprise entre 0° C et la température ambiante, en présence d'une base telle que la triéthylamine.

Une variante au mode opératoire consiste à préparer l'anhydride mixte de l'acide de formule (II) par réaction du chloroformiate d'éthyle avec l'acide de formule (II), en présence d'une base telle que la triéthylamine, et à le faire réagir avec la 1-aminopipéridine, dans un solvant tel que le dichlorométhane, sous une atmosphère inerte, à la température ambiante, en présence d'une base telle que la triéthylamine.

Le composé de formule (I) ainsi obtenu est isolé, sous forme de base libre ou de sel ou de solvate, selon les techniques conventionnelles.

Le composé de formule (I) peut être isolé sous forme d'un de ses sels, par exemple le chlorhydrate ou l'oxalate ; dans ce cas, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée dans un autre sel comme le méthanesulfonate, le fumarate ou le 2-naphtalènesulfonate.

Lorsque le composé (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

L'acide de formule (II) utilisé comme composé de départ pour le procédé de la présente invention peut être préparé selon des méthodes classiques. Certaines de ces méthodes sont illustrées en détail ci-après dans les PREPARATIONS.

Les préparations 1 et 2 sont voisines. Elles sont effectuées selon le schéma réactionnel décrit ci-après (Voie 1).

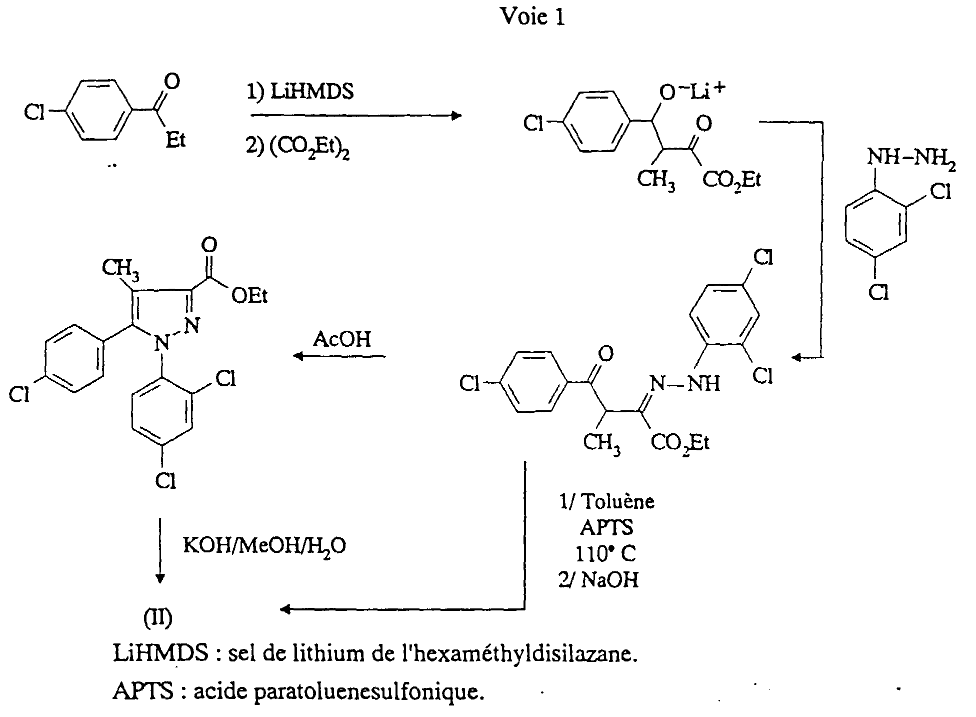

La première étape est effectuée d'après J. Heterocyclic. Chem., 1989, 26, 1389.

La préparation 3 est réalisée selon le schéma décrit ci-après (voie 2).

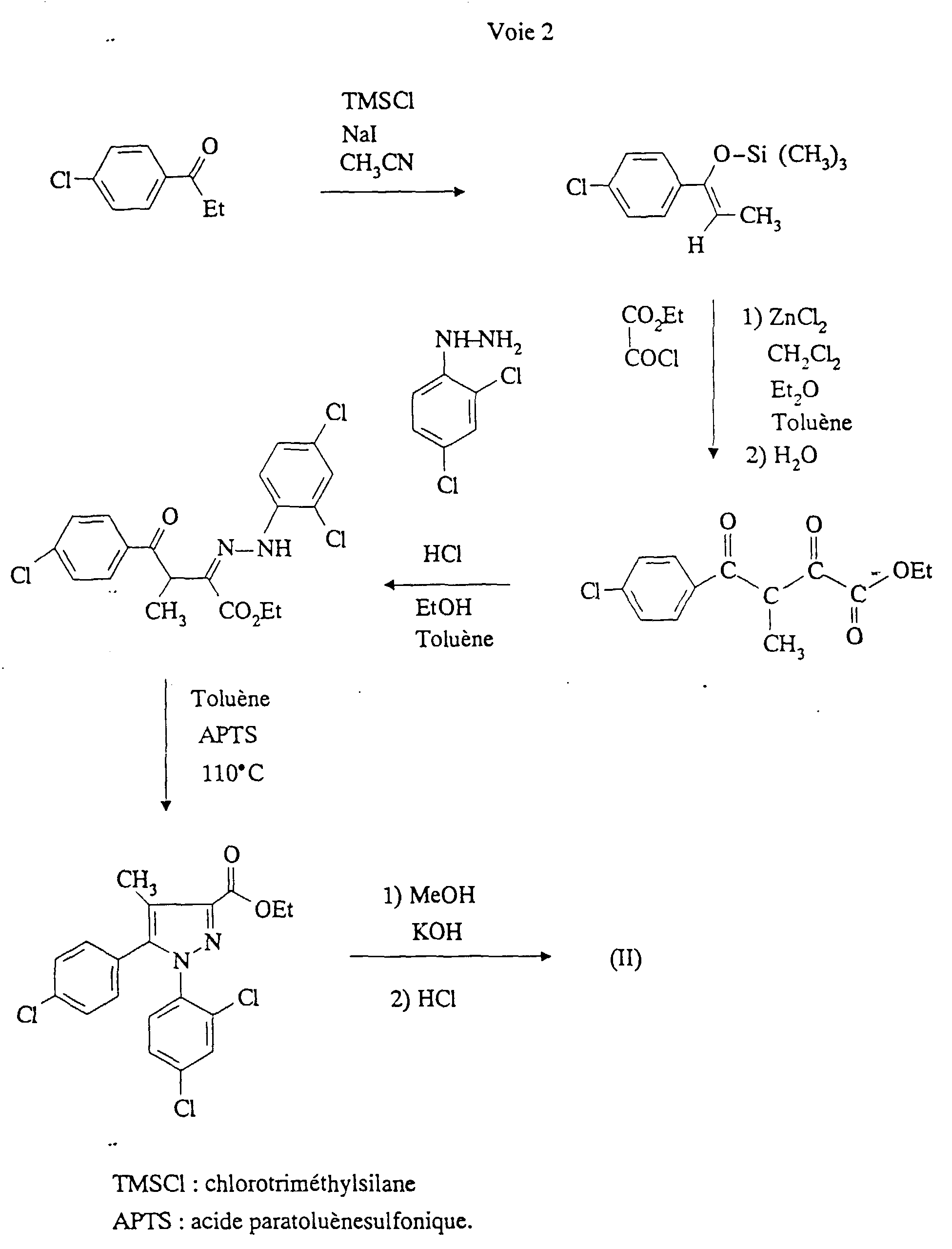

La première étape est réalisée d'après E.S. SCHWEIZER, J. Org. Chem. 1987, 52, 1324-1332. La seconde étape est réalisée d'après R.E. TIRPAK et al., J. Org. Chem., 1982, 47, 5099-5102.

L'autre réactif utilisé pour le procédé de la présente invention, la 1-aminopipéridine, est un produit commercial.

Le composé de formule (I) possède une très bonne affinité *in vitro* pour les récepteurs aux cannabinoides centraux, dans les conditions expérimentales décrites par Devane et al., Molecular Pharmacology, 1988, 34, 605-613.

Plus particulièrement, le composé de la présente invention, tel quel ou sous forme d'un de ses sels pharmaceutiquement acceptables, est un antagoniste puissant et sélectif des récepteurs aux cannabinoïdes centraux, ayant un Ki de 2 nM environ. Il est entre 500 et 1000 fois plus actif sur le récepteur central que sur le récepteur périphérique, est actif par voie orale et passe la barrière hématoencéphalique.

La bonne pénétration du composé de la présente invention dans le système nerveux central, ainsi que sa nature antagoniste, sont confirmées par les résultats dans le modèle de l'antagonisme de l'hypothermie provoquée par un agoniste des récepteurs aux cannabinoïdes. Notamment le composé de la présente invention antagonise l'hypothermie induite par le WIN 55212-2 chez la souris avec une DE₅₀ de 0,3 mg/kg i.p. et de 0,4 mg/kg per os ; dans ce test (Pertwee R.G. dans Marijuana 84, Ed. Harvey, D.Y., Oxford IRL Press, 1985, 263-277), le composé a montré une durée d'action de 8 à 10 heures après administration orale d'une dose de 3 mg/kg.

De plus, le composé (I) administré seul, par voie sous-cutanée améliore les capacités mnésiques du rat dans le test de la mémoire centrale (A. Pério et al., Psychopharmacology, 1989, 97, 262-268).

Grâce à ses remarquables propriétés, notamment à sa grande affinité, à sa sélectivité pour le récepteur central et à sa capacité de pénétrer la barrière hématoencéphalique, le composé (I), tel quel ou éventuellement sous forme de sel pharmaceutiquement acceptable ou de solvate, peut être utilisé comme principe actif de médicaments destinés à combattre les maladies du système nerveux central des mammifères.

La toxicité du composé (I) est compatible avec son utilisation en tant que médicament psychotrope, notamment pour le traitement des troubles thymiques, des troubles anxieux, des troubles de l'humeur, du vomissement, des troubles mnésiques, des troubles cognitifs, des neuropathies, de la migraine, du stress, des maladies d'origine psychosomatique, de l'épilepsie, des dyskinésies ou de la maladie de Parkinson.

Le composé (I) selon l'invention peut également être utilisé en tant que médicament pour le traitement des troubles de l'appétit notamment en tant qu'anorexigène, pour le traitement de la schizophrénie, des troubles délirants, des troubles psychotiques en général, ainsi que des troubles liés à l'utilisation de substances psychotropes. De plus, le composé (I) selon l'invention peut être utilisé en tant que médicament pour la chimiothérapie anticancéreuse.

L'utilisation du composé selon l'invention en tant que médicament pour le traitement des troubles de l'appétit, des troubles anxieux, des troubles de l'humeur, de la schizophrénie, des troubles psychotiques, des troubles mnésiques, des troubles cognitifs et des dyskinésies, ainsi que son utilisation dans la chimiothérapie anticancéreuse, constituent un aspect ultérieur de la présente invention.

Le composé selon l'invention est généralement administré en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables ou un de leurs solvates.

Le composé de formule (I) ci-dessus et ses sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration orale, sublinguale, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Dans les compositions pharmaceutiques de la présente invention, le principe actif est généralement formulé en unités de dosage contenant de 0,5 à 1000 mg, avantageusement de 1 à 500 mg, de préférence de 2 à 200 mg dudit principe actif par unité de dosage pour les administrations quotidiennes.

Lorsque l'on prépare une composition solide sous forme de comprimé, on peut ajouter au principe actif micronisé ou non un agent mouillant tel que le laurylsulfate de sodium et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse on peut utiliser un cosolvant : un alcool tel que l'éthanol, un glycol tel que le polyéthylèneglycol ou le propylèneglycol et un tensioactif hydrophile tel que le Tween® 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminé ou des réservoirs dans lequel le principe actif est en solution alcoolique.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ- cyclodextrine,2-hydroxypropyl-β-cyclodextrine ou méthyl-β-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Les exemples suivants illustrent l'invention sans toutefois la limiter.

Les points de fusion ou de décomposition des produits, F, ont été mesurés en tube capillaire avec un appareil de Tottoli ; dans certains cas on a utilisé l'analyse calorimétrique différentielle (DSC) pour mesurer la température de fusion.

Dans la préparation et dans les exemples, les abréviations suivantes sont utilisées:
THF : tétrahydrofurane
Ether : éther diéthylique
Ether iso : éther diisopropylique
EtOH : : éthanol
AcOEt : acétate d'éthyle
MeOH : méthanol
DCM : dichlorométhane
KOH : potasse
AcOH : acide acétique
HCl : acide chlorhydrique
NaCl : chlorure de sodium
TA : température ambiante
DSC : analyse calorimétrique différentielle
F : point de fusion

Pour l'interprétation des spectres RMN on utilise les abréviations suivantes :
s : singulet
d : doublet
t : triplet
q : quadruplet
m : multiplet ou massif.

### Préparation 1

### A) Sel de lithium du 4-(4-chlorophényl)-3-méthyl-4-oxydo-2-oxobut-3-énoate d'éthyle.

On ajoute, sous atmosphère d'azote, 125 ml d'une solution 1M du sel de lithium de l'hexaméthyldisilazane dans le THF, à 500 ml d'éther. On refroidit à -78°C et ajoute, goutte à goutte, une solution de 21 g de 4-chloropropiophénone dans 100 ml d'éther. Après 45 minutes d'agitation, on ajoute rapidement 19,2 ml d'oxalate d'éthyle et laisse 16 heures sous agitation en laissant remonter la température à TA. On filtre le précipité formé, lave à l'éther et le sèche sous vide. On obtient 12,6 g du produit attendu.

### B) Ester éthylique de l'acide 5-(4-chlorophényl)-1-(2,4-dichlorophényl)-4-méthylpyrazole-3-carboxylique.

A une solution de 12,6 g du sel de lithium obtenu à l'étape précédente dans 70 ml d'EtOH on ajoute 9,8 g de 2,4-dichlorophénylhydrazine et laisse 16 heures sous agitation à TA. On filtre le précipité formé, lave à l'EtOH puis à l'éther et sèche sous vide. On obtient 12,6 g d'hydrazone que l'on dissout dans 100 ml d'AcOH. On chauffe à reflux pendant 24 heures puis verse la mélange réactionnel dans 500 ml d'eau glacée. On extrait à l'AcOEt, lave à l'eau, par une solution saturée de NaCl, sèche sur sulfate de magnésium et évapore sous vide. On obtient le produit attendu après cristallisation dans l'éther iso, m= 9,6 g, F = 124°C.

### C) Acide 5-(4-chlorophényl)-1-(2,4-dichlorophényl)-4-méthylpyrazole-3-carboxylique.

A une solution de 9,6 g de l'ester obtenu à l'étape précédente dans 70 ml de MeOH on ajoute une solution de 3,3 g de KOH dans 70 ml d'eau et chauffe à reflux pendant 3 heures. On verse le mélange réactionnel sur 200 ml d'eau glacée, acidifie à pH = 1 par ajout d'une solution à 10 % d'HCl, filtre le précipité formé, lave à l'eau et sèche sous vide. On obtient 8,8 g de l'acide attendu, F = 211°C.

La préparation 1 est améliorée en utilisant les conditions opératoires décrites dans la préparation 2 ci-après.

### Préparation 2

### A) Sel de lithium du 4-(4-chlorophényl)-3-méthyl-4-oxydo-2-oxobut-3-énoate d'éthyle.

Sous atmosphère d'azote, dans un réacteur, on met en solution 2008 g du sel de lithium de l'hexaméthyldisilazane dans 10,1 litres de méthylcyclohexane. On ajoute lentement à 20°C ± 5°C, une solution de 1686 g de 4-chloropropiophénone dans 4 litres de méthylcyclohexane. Après 4 heures 30 minutes d'agitation, on ajoute à 20°C ± 5°C en 35 minutes 1607 g d'oxalate d'éthyle. On laisse 17 heures sous agitation à la même température. On filtre le solide formé, lave au méthylcyclohexane et sèche sous vide (masse de produit obtenu: 1931 g).

### B) Acide 5-(4-chlorophényl)-1-(2,4-dichlorophényl)-4-méthylpyrazole-3-carboxylique.

1) Sous atmosphère d'azote, dans un réacteur, on met en solution 1921 g du sel de lithium obtenu à l'étape précédente dans 11 litres d'éthanol. A 20°C ± 5°C, on ajoute immédiatement 1493 g de chlorhydrate de 2,4-dichlorophénylhydrazine. On agite pendant 1 heure puis ajoute 2,88 litres d'eau désionisée et on maintient l'agitation 17 heures à 20°C ± 5°C. On filtre le précipité formé, lave à l'éthanol à 80 % (vol/vol) et sèche sous vide (masse d'hydrazone obtenue = 2280 g), F = 140°C.
2) Sous atmosphère d'azote, dans un réacteur on met, en solution 2267 g d'hydrazone dans 11,3 litres de toluène. On ajoute 201,6 g d'acide paratoluènesulfonique et chauffe au reflux du toluène pendant 3 heures. On refroidit à 20°C ± 5°C et élimine l'acide paratoluènesulfonique par extraction à l'eau désionisée. A la solution toluènique, on ajoute 120,75 g de chlorure de benzyltriéthylammonium et 636 g de NaOH en solution dans 1180 ml d'eau désionisée. Sous bonne agitation, on chauffe 4 heures à 68°C ± 3°C, puis on neutralise l'hydroxyde de sodium et acidifie le mélange réactionnel avec 1500 ml d'HCl (d = 1,19). On refroidit à 20°C ± 5°C, filtre le précipité formé, lave au toluène, à l'eau désionisée et sèche sous vide (masse de produit obtenu : 1585 g), F = 210°C.

### Préparation 3

### A)1-(4-chlorophényl)-1-triméthylsilyloxypropéne.

Sous atmosphère d'azote à 20°C ± 3°C, on ajoute lentement 13,47 g de chlorotriméthylsilane à 12,55 g de triéthylamine. On poursuit l'addition des réactifs en ajoutant 16,86 g de 4-chloropropiophénone (mélange endothermique) puis une solution de 18,58 g d'iodure de sodium dans 125 ml d'acétonitrile tout en maintenant la température à 18°C ± 2°C. On chauffe ensuite pendant 3 heures à 40°C ± 5°C. On élimine l'acétonitrile sous pression réduite et ajoute au résidu solide 150 ml de toluène. On distille sous pression réduite 50 ml de solvant afin d'entrainer l'acétonitrile résiduel. On extrait les minéraux avec 100 ml d'eau glacée, lave la phase organique avec 100 ml d'eau glacée et sèche sur sulfate de magnésium. On élimine le toluène sous pression réduite et on termine l'élimination des solvants à 60°C sous une pression de 1mbar pendant 15 heures (masse de l'huile obtenue : 22,70 g).
RMN à 200 MHz (CDCl₃)
0,13 ppm : s : 9H
1,7 ppm : d : 3H
5,28 ppm : q : 1H
7,21-7,39 ppm : m : 4H

### B) 3-(4-chlorobenzoyl)-3-méthylpyruvate d'éthyle.

Sous atmosphère d'azote on met en suspension 10 g de chlorure de zinc anhydre dans 50 ml de toluène. On élimine l'eau résiduelle par entrainement azéotropique à pression atmosphérique en 1 heure. On refroidit à 20°C ± 3°C, ajoute 20 ml de toluène et 11,5 ml d'éther éthylique. A 0°C ± 2°C, on additionne lentement 17,0 g de chloroglyoxylate d'éthyle dilué dans 20 ml de dichlorométhane. A la même température on ajoute en 1 heure 30 minutes 14,5 g du produit obtenu à l'étape précédente. On laisse remonter la température à TA puis on chauffe à 45° C pendant 4 heures. On lave la phase organique par une solution d'hydrogénocarbonate de sodium, par l'eau et sèche sur sulfate de magésium. On élimine les solvants sous pression réduite (masse d'huile obtenue : : 17,6 g).
RMN à 200 MHz (CDCl₃)
1,25 ppm : t : 3H
1,35ppm : d : 3H
4,20 ppm : q : 2H
4,93 ppm : q : 1H
7,45-7,90 ppm : m : 4H

### C) Ester éthylique de l'acide 5-(4-chlorophényl)-1-(2,4-dichlorophényl)-4-méthylpyrazole-3-carboxylique.

A 17,6 g du composé obtenu à l'étape précédente, on ajoute 13,3 g de chlorhydrate de 2,4-dichlorophénylhydrazine et laisse 18 heures sous agitation à 20°C ± 3°C. Sans isoler l'hydrazone, on ajoute 0,56 g d'acide paratoluènesulfonique et distille l'azéotrope ternaire (eau, éthanol, toluène). On maintient au reflux du toluène une heure. On refroidit le mélange réactionnel à 20°C ± 3°C, filtre l'insoluble puis lave la solution toluénique avec 2 fois 100 ml d'eau. On élimine les solvants sous pression réduite (l'huile brute est utilisée telle quelle dans l'étape suivante).

### D) Acide 5-(4-chlorophényl)-1-(2,4-dichlorophényl)-4-méthylpyrazole-3-carboxylique.

A une solution de l'huile obtenue à l'étape précédente dans 100 ml de MeOH, on ajoute 8,1 g de KOH en pastilles. Après 1 heure à 25°C ± 3°C, on élimine les solvants sous pression réduite. On reprend le résidu avec 200 ml d'eau et 40 ml de toluène. On chauffe à 60°C ± 3°C, décante et extrait à cette température la phase aqueuse avec 3 fois 40 ml de toluène. On ajoute à la phase aqueuse de l'acide chlorhydrique jusqu'à pH = 1,5. On filtre les cristaux blancs formés, lave à l'eau et à l'éther iso et sèche sous vide (masse de produit obtenu : 9,9 g), F = 210°C.

### EXEMPLE 1

N-Pipéridino-5-(4-chlorophényl)-1-(2,4-dichlorophényl)-4-méthylpyrazole-3-carboxamide.

### A) Chlorure de l'acide 5-(4-chlorophényl)-1-(2,4-dichlorophényl)-4-méthylpyrazole-3-carboxylique.

A une suspension de 8,8 g de l'acide obtenu à l'étape C de la préparation 1 dans 90 ml de toluène on ajoute 5 ml de chlorure de thionyle et chauffe à reflux pendant 3 heures. On évapore sous vide à siccité le mélange réactionnel, reprend le résidu dans 90 ml de toluène et reévapore sous vide. On obtient 8,0 g de chlorure d'acide attendu qui est utilisé tel quel à l'étape suivante.

### B) N-Pipéridino-5-(4-chlorophényl)-1-(2,4-dichlorophényl)-4-méthylpyrazole-3-carboxamide.

On refroidit à 0°C une solution de 2,8 ml de 1-aminopipéridine, 3,6 ml de triéthylamine dans 100 ml de DCM et ajoute, goutte à goutte, une solution de 8,0 g de chlorure d'acide obtenu à l'étape précédente dans 80 ml de DCM. On laisse 3 heures sous agitation en laissant remonter la température à TA puis verse sur 200 ml d'eau glacée. On extrait au DCM, lave à l'eau, par une solution saturée de NaCl, sèche sur sulfate de magnésium et évapore sous vide. On chromatographie sur silice en éluant par le mélange AcOEt/toluène (10/90 ; v/v). On obtient le produit attendu après cristallisation dans l'éther iso, m = 5,9 g, F = 148°C.

On peut également préparer le composé de l'exemple 1 en utilisant des conditions opératoires plus accessibles de façon industrielle.

Sous atmosphère d'azote, à une suspension de 1568,6 g de l'acide obtenu à l'étape B de la préparation 2 dans 14,1 litres de méthylcyclohexane, on ajoute après chauffage à 83°C ± 3°C, une solution de 554,5 g de chlorure de thionyle dans 1,57 litres de méthylcyclohexane. On agite 3 heures à 83°C ± 3°C puis on élève la température du mélange réactionnel en 2 heures jusqu'au reflux du méthylcyclohexane tout en éliminant l'excès de chlorure de thionyle par distillation. On refroidit à 10°C ± 3°C et ajoute lentement une solution de 452,9 g de 1-aminopipéridine et de 457,5 g de triéthylamine dans 3,14 litres de THF. On agite 17 heures en laissant remonter la température à 20°C ± 5°C puis on lave successivement la phase organique à 20°C ± 5°C par l'eau désionisée et l'acide acétique à 4 % dans l'eau, on termine les lavages de la phase organique à 70°C ± 3°C par une solution de NaOH à 1,5 %, puis par l'eau désionisée et on entraine le THF et l'eau par distillation azéotropique à pression atmosphérique. On laisse refroidir à 20°C ± 5°C. Le produit attendu cristallise. On filtre le précipité formé, lave au méthylcyclohexane et sèche sous vide (masse de produit obtenu : 1627 g).

DSC : pic endothermique centré à 155,5°C.

### EXEMPLE 2

N-pipéridino-5-(4-chlorophényl)-1-(2,4-dichlorophényl)-4-méthylpyrazole-3-carboxamide (solvate avec éthanol).

On met en suspension 10 g du composé obtenu à l'exemple 1 dans 60 ml d'éthanol absolu et on chauffe au reflux jusqu'à dissolution complète. On laisse refroidir à 20°C ± 3°C et maintient l'agitation 2 heures. On filtre les cristaux blancs formés, lave à l'éthanol et sèche sous vide (masse de produit obtenu : 9,60 g).

DSC : pic endothermique centré à 102,7°C

| | | | |
|---|---|---|---|
| % calculés | C = 56,5 | H = 5,29 | N = 10,98 |
| % trouvés | C = 56,43 | H = 5,41 | N = 11,05 |

### EXEMPLE 3

Chlorhydrate de N-pipéridino-5-(4-chlorophényl)-1-(2,4-dichlorophényl)-4-méthylpyrazole-3-carboxamide.

A une solution de 5,9 g du composé obtenu à l'EXEMPLE 1 dans 50 ml d'éther, on ajoute, goutte à goutte, une solution saturée d'HCl gazeux dans l'éther jusqu'à pH = 1. On filtre le précipité formé, lave à l'éther et le sèche sous vide.. On obtient 6,0 g du chlorhydrate attendu, F = 224°C (décomposition).

### EXEMPLE 4

Chlorhydrate de N-pipéridino-5-(4-chlorophényl)-1-(2,4-dichlorophényl)-4-méthylpyrazole-3-carboxamide (solvate avec éthanol).

On met en suspension 40 g du composé obtenu à l'exemple 3 dans 400 ml d'éthanol absolu. On chauffe à l'ébullition jusqu'à dissolution complète et on laisse 20 heures en agitation en refroidissant progessivement à 20°C ± 3°C. On filtre les cristaux blancs formés, lave à l'éthanol, sèche sous vide (masse de produit obtenu : 29,6 g).

DSC : massif endothermique très étalé (175 à 230°C). Thermogravimétrie : perte de masse (8,2 % environ) à partir de 100°C.

| | | | |
|---|---|---|---|
| % calculés | C = 53,04 | H = 5,16 | N = 10,31 |
| % trouvés | C = 52,68 | H = 5,23 | N = 10,34 |

### EXEMPLE 5

Méthanesulfonate de N-pipéridino-5-(4-chlorophényl)-1-(2,4-dichlorophényl)-4-méthylpyrazole-3-carboxamide (hémisolvate avec acétone).

A une solution de 18,55 g de composé obtenu à l'exemple 1 dans 185 ml d'acétone, on ajoute à 20°C ± 3°C, 3,84 g d'acide méthanesulfonique et on agite 20 heures à la même température. On filtre les cristaux blancs formés, lave à l'acétone et sèche sous vide (masse de produit obtenu : 21,65 g).

DSC : fusion, recristallisation vers 175°C puis fusion à 191,5°C

Thermogravimétrie : perte de masse (5,2 % environ) à partir de 90°C.

| | | | |
|---|---|---|---|
| % calculés | C = 49,90 | H = 4,75 | N = 9,5 |
| % trouvés | C = 49,70 | H = 4,76 | N = 9,44 |

### EXEMPLE 6

Hémifumarate de N-pipéridino-5-(4-chlorophényl)-1-(2,4-dichlorophényl)-4-méthylpyrazole-3-carboxamide.

A une solution de 0,30 g de composé obtenu à l'EXEMPLE 1 dans 3 ml d'acétone, on ajoute goutte à goutte une solution de 0,038 g d'acide fumarique dans 6 ml d'acétone. On filtre les cristaux blancs formés par refroidissement à 0°C, lave à l'acétone et sèche sous vide. On obtient 0,23 g du sel attendu, F = 168°C.

### EXEMPLE 7

Hydrogénosulfate de N-pipéridino-5-(4-chlorophényl)-1-(2,4-dichlorophényl)-4-méthylpyrazole-3-carboxamide.

A une solution de 0,30 g de composé obtenu à l'EXEMPLE 1 dans 3 ml d'acétone, on ajoute 0,018 ml d'acide sulfurique concentré. On filtre les cristaux blancs formés, lave à l'acétone et à l'éther et sèche sous vide. On obtient 0,31 g du sel attendu, F = 240°C.

### EXEMPLE 8

Paratoluènesulfonate de N-pipéridino-5-(4-chlorophényl)-1-(2,4-dichlorophényl)-4-méthylpyrazole-3-carboxamide.

A une solution de 18,55 g du composé obtenu à l'exemple 1 dans 185 ml d'acétone, on ajoute à 20°C ± 3°C, 7,61 g d'acide paratoluènesulfonique et on agite 20 heures à la même température. On filtre les cristaux blancs formés, lave à l'acétone et sèche sous vide (masse de produit obtenu : 24,25 g).

DSC : pic endothermique centré à 236,8°C

| | | | |
|---|---|---|---|
| % calculés | C = 54,76 | H = 4,60 | N = 8,72 |
| % trouvés | C = 54,11 | H = 4,71 | N = 8,69 |

### EXEMPLE 9

Dihydrogénophosphate de N-pipéridino-5-(4-chlorophényl)-1-(2,4-dichlorophényl)-4-méthylpyrazole-3-carboxamide.

A une solution de 18,55 g du composé obtenu à l'exemple 1 dans 185 ml de méthyléthylcétone, on ajoute à 20°C ± 3°C, 4,61 g d'acide phosphorique à 85 %. On élimine l'eau par distillation à pression atmosphérique de l'azéotrope méthyléthylcétone-eau. On refroidit progressivement à 20°C ± 3°C tout en maintenant l'agitation 20 heures. On filtre les cristaux blancs formés, lave à la méthyléthylcétone et sèche sous vide (masse de produit obtenu : 21,0 g).

DSC : pic endothermique centré à 185,5°C

| | | | |
|---|---|---|---|
| % calculés | C = 47,04 | H = 4,31 | N = 9,97 |
| % trouvés | C = 46,96 | H = 4,62 | N = 9,98 |

## Revendications

1. Le N-pipéridino-5-(4-chlorophényl)-1-(2,4-dichlorophényl)-4-méthylpyrazole-3-carboxamide de formule : 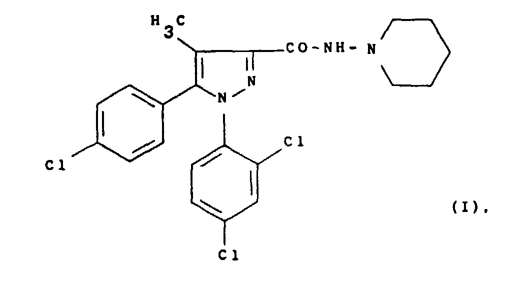 ses sels d'addition d'acides pharmaceutiquement acceptables et leurs solvates.

2. Composé selon la revendication 1, qui est le chlorhydrate de N-pipéridino-5-(4-chlorophényl)-1-(2,4-dichlorophényl)-4-méthylpyrazole-3-carboxamide ou son solvate avec l'éthanol.

3. Composé selon la revendication 1, qui est le méthanesulfonate de N-pipéridino-5-(4-chlorophényl)-1-(2,4-dichlorophényl)-4-méthylpyrazole-3-carboxamide ou son hémisolvate avec l'acétone.

4. Composé selon la revendication 1, qui est l'hemifumarate de N-pipéridino-5-(4-chlorophényl)-1-(2,4-dichlorophényl)-4-méthylpyrazole-3-carboxamide.

5. Composé selon la revendication 1, qui est le paratoluènesulfonate de N-pipéridino-5-(4-chlorophényl)-1-(2,4-dichlorophényl)-4-méthylpyrazole-3-carboxamide.

6. Composé selon la revendication 1, qui est l'hydrogénosulfate de N-pipéridino-5-(4-chlorophényl)-1-(2,4-dichlorophényl)-4-méthylpyrazole-3-carboxamide.

7. Composé selon la revendication 1, qui est le dihydrogénophosphate de N-pipéridino-5-(4-chlorophényl)-1-(2,4-dichlorophényl)-4-méthylpyrazole-3-carboxamide.

8. Procédé pour la préparation du N-pipéridino-5-(4-chlorophényl)-1-(2,4-dichlorophényl)-4-méthylpyrazole-3-carboxamide, de ses sels et de leurs solvates, caractérisé en ce qu'on traite un dérivé fonctionnel de l'acide 5-(4-chlorophényl)-1-(2,4-dichlorophényl)-4-méthylpyrazole-3-carboxylique de formule : 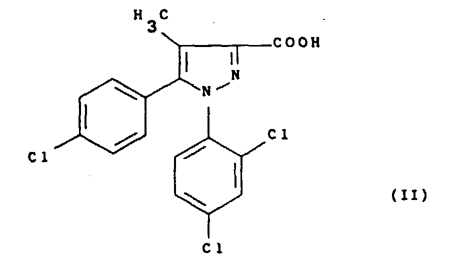 avec la 1-aminopipéridine dans un solvant organique et en présence d'une base, et éventuellement on transforme le composé ainsi obtenu en l'un de ses sels ou l'un de leurs solvates.

9. Composition pharmaceutique contenant, en tant que principe actif, un composé selon l'une quelconque des revendications 1 à 7.

10. Composition pharmaceutique selon la revendications 9, sous forme d'unité de dosage, dans laquelle le principe actif est mélangé à au moins un excipient pharmaceutique.

11. Composition pharmaceutique selon la revendication 10, contenant de 0,5 à 1000 mg de principe actif.

## Patentansprüche

1. N-Piperidino-5-(4-chlorphenyl)-1-(2,4-dichlorphenyl)-4-methylpyrazol-3-carboxamid der Formel: 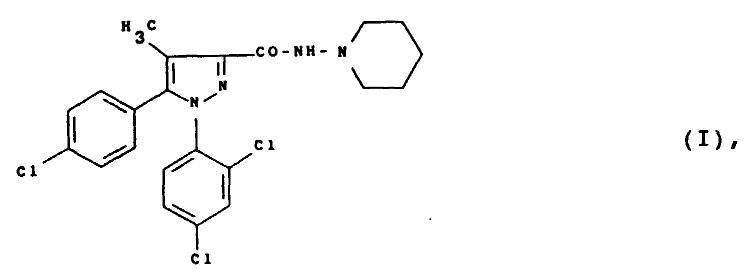 seine pharmazeutisch annehmbaren Säureadditionssalze und deren Solvate.

2. Verbindung nach Anspruch 1, welche das Chlorhydrat von N-Piperidino-5-(4-chlorphenyl)-1-(2,4-dichlorphenyl)-4-methylpyrazol-3-carboxamid oder sein Solvat mit Ethanol ist.

3. Verbindung nach Anspruch 1, welche das Methansulfonat von N-Piperidino-5-(4-chlorphenyl)-1-(2,4-dichlorphenyl)-4-methylpyrazol-3-carboxamid oder sein Hemisolvat mit Aceton ist.

4. Verbindung nach Anspruch 1, welche das Hemifumarat von N-Piperidino-5-(4-chlorphenyl)-1-(2,4-dichlorphenyl)-4-methylpyrazol-3-carboxamid ist.

5. Verbindung nach Anspruch 1, welche das p-Toluolsulfonat von N-Piperidino-5-(4-chlorphenyl)-1-(2,4-dichlorphenyl)-4-methylpyrazol-3-carboxamid ist.

6. Verbindung nach Anspruch 1, welche das Hydrogensulfat von N-Piperidino-5-(4-chlorphenyl)-1-(2,4-dichlorphenyl)-4-methylpyrazol-3-carboxamid ist.

7. Verbindung nach Anspruch 1, welche das Dihydrogenphosphat von N-Piperidino-5-(4-chlorphenyl)-1-(2,4-dichlorphenyl)-4-methylpyrazol-3-carboxamid ist.

8. Verfahren zur Herstellung von N-Piperidino-5-(4-chlorphenyl)-1-(2,4-dichlorphenyl)-4-methylpyrazol-3-carboxamid, seiner Salze und deren Solvate, dadurch gekennzeichnet, daß ein funktionelles Derivat von 5-(4-Chlorphenyl)-1-(2,4-dichlorphenyl)-4-methylpyrazol-3-carbonsäure der Formel: 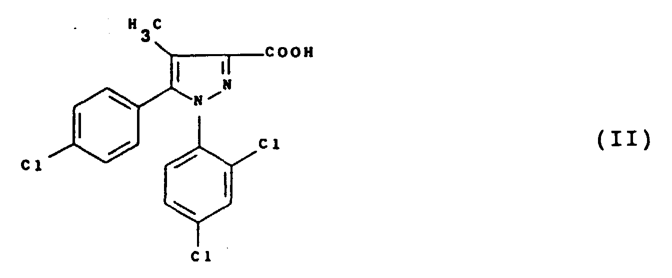 mit 1-Aminopiperidin in einem organischen Lösungsmittel und in Anwesenheit einer Base behandelt wird, und gegebenenfalls die so erhaltene Verbindung in eines ihrer Salze oder eines deren Solvate transformiert wird.

9. Pharmazeutische Zusammensetzung, welche als aktiven Bestandteil eine Verbindung nach einem der Ansprüche 1 bis 7 enthält.

10. Pharmazeutische Zusammensetzung nach Anspruch 9 in Form einer Dosierungseinheit, worin der aktive Bestandteil mit zumindest einem pharmazeutischen Exzipienten gemischt ist.

11. Pharmazeutische Zusammensetzung nach Anspruch 10, welche 0,5 bis 1000 mg aktiven Bestandteil enthält.

## Claims

1. N-piperidino-5-(4-chlorophenyl)-1-(2,4-dichlorophenyl)-4-methylpyrazole-3-carboxamide of the formula : 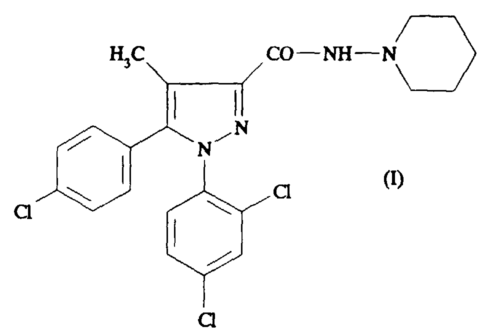 its pharmaceutically acceptable acid addition salts and the solvates thereof.

2. A compound according to claim 1, which is N-piperidino-5-(4-chlorophenyl)-1-(2,4-dichlorophenyl)-4-methylpyrazole-3-carboxamide hydrochloride or its solvate with ethanol.

3. A compound according to claim 1, which is N-piperidino-5-(4-chlorophenyl)-1-(2,4-dichlorophenyl)-4-methylpyrazole-3-carboxamide methanesulfonate or its hemisolvate with acetone.

4. A compound according to claim 1, which is N-piperidino-5-(4-chlorophenyl)-1-(2,4-dichlorophenyl)-4-methylpyrazole-3-carboxamide hemifumarate.

5. A compound according to claim 1, which is N-piperidino-5-(4-chlorophenyl)-1-(2,4-dichlorophenyl)-4-methylpyrazole-3-carboxamide *p*-toluenesulfonate.

6. A compound according to claim 1, which is N-piperidino-5-(4-chlorophenyl)-1-(2,4-dichlorophenyl)-4-methylpyrazole-3-carboxamide hydrogensulfate.

7. A compound according to claim 1, which is N-piperidino-5-(4-chlorophenyl)-1-(2,4-dichlorophenyl)-4-methylpyrazole-3-carboxamide dihydrogenphosphate.

8. A process for the preparation of N-piperidino-5-(4-chlorophenyl)-1-(2,4-dichlorophenyl)-4-methylpyrazole-3-carboxamide, its salts and their solvates, characterized in that a functional derivative of 5-(4-chlorophenyl)-1-(2,4-dichlorophenyl)-4-methylpyrazole-3-carboxylic acid of the formula: 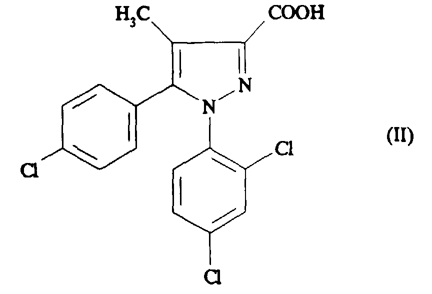 is treated with 1-aminopiperidine in an organic solvent in the presence of a base, and, the resulting compound is optionally converted into one of its salts or a solvate thereof.

9. A pharmaceutical composition, which contains a compound according to any one of claims 1 to 7 as the active principle.

10. A pharmaceutical composition according to claim 9, which is in dosage unit form, wherein the active principle is mixed with at least one pharmaceutical excipient.

11. A pharmaceutical composition according to claim 10, which contains from 0.5 to 1,000 mg of active principle.
